# EUROPÄISCHE PATENTSCHRIFT

(11) **EP 3 709 969 B1**
(45) Veröffentlichungstag und Bekanntmachung des Hinweises auf die Patenterteilung: **23.08.2023**
(21) Anmeldenummer: 18799567.5
(22) Anmeldetag: 14.11.2018
(51) Int. Cl.: A61K 9/00, A61Q 7/00, A61K 45/06, A61K 47/10, A61K 47/18, A61K 9/06, A61K 9/107, A61K 31/14, A61K 31/192, A61K 31/196, A61K 31/496, A61K 31/665, A61K 31/7056, A61K 31/4706, A61K 31/4174, A61P 15/02, A61P 29/00, A61P 31/04, A61P 31/10

(54) **EMULSIONEN ZUR TOPISCHEN BEHANDLUNG VON DERMALEN INFEKTIONEN UND SCHLEIMHAUTINFEKTIONEN**
EMULSIONS FOR THE TOPICAL TREATMENT OF DERMAL AND MUCOSAL INFECTIONS
ÉMULSIONS DE TRAITEMENT TOPIQUE D'INFECTIONS DERMIQUES ET MUQUEUSES

(30) Priorität: 14.11.2017 EP 17201651; 14.11.2017 EP 17201650
(43) Veröffentlichungstag der Anmeldung: 23.09.2020
(73) Patentinhaber: ProFem GmbH, 1180 Wien (AT)
(72) Erfinder: NOE, Marion, 1180 Wien (AT); NOE, Christian, 1180 Wien (AT)
(74) Vertreter: SONN Patentanwälte OG
(86) Internationale Anmeldenummer: PCT/EP2018/081264
(87) Internationale Veröffentlichungsnummer: WO 2019/096863

(56) Entgegenhaltungen:
- EP-A2- 0 923 937
- WO-A1-2011/060083
- WO-A2-02/078648
- WO-A2-2007/131253
- AT-U1- 11 910
- DE-A1-102008 034 944
- US-A- 5 686 089
- US-A1- 2007 292 355

## Beschreibung

Die Erfindung betrifft Emulsionen zur topischen Behandlung von dermalen Infektionen und Schleimhautinfektionen, insbesondere von urogenitalen Infektionen.

Der Mensch lebt in Symbiose mit einer gigantischen Zahl von Mikroorganismen, welche vor allem seinen Darm und seine Hautoberfläche als Kommensalen besiedeln. Die Gesamtheit dieser Mikroorganismen wird als menschliches Mikrobiom bezeichnet. Eine ganz spezielle Zusammensetzung besitzt das Mikrobiom der Scheide der Frau, in welchem Milchsäurebakterien vorherrschen. Diese erzeugen in der Scheide unter normalen physiologischen Bedingungen ein schwach saures Milieu, das protektiv gegen bakterielle Infektionen wirkt. Dennoch treten aus verschiedenen Ursachen häufig Vaginalinfektionen auf, verursacht einerseits etwa durch die fast ubiquitär vorhandenen Pilze der Spezies Candida, welche sich leicht von Kommensalen zu Pathogenen wandeln, andererseits durch Bakterien wie Gardnerella vaginalis, Mobiluncus oder Prevotella spp., sowie durch Streptokokken oder Staphylokokken oder etwa durch typische Darmkeime, wie Enterobacter, E. coli und/oder etwa Klebsiella pneumoniae , welche alleine schon wegen der räumlichen Nähe der Körperöffnungen leicht durch Schmierinfektion in die Scheide gelangen können. Zunächst sind asymptomatische Fehlbesiedelungen die Folge. Wenn bestimmte Schwellenwerte an Fremdkeimen überschritten sind und infektionsunterstützende Faktoren hinzutreten, bricht letztlich ein virulentes infektiöses Geschehen aus. Wenn dieses nicht rechtzeitig behandelt wird, kann es in der Folge meist unter Biofilmbildung zur Chronifizierung der Infektion kommen. Schon asymptomatische Fehlbesiedelungen werden zunehmend auch als Risikofaktoren für Unfruchtbarkeit, Fehlgeburtlichkeit und Frühgeburtlichkeit aber auch bei Blasenentzündungen und bestimmten Formen der Inkontinenz erkannt.

Die US 2007/292355 A1 betrifft antiinfektiöse schäumbare Zusammensetzungen enthaltend ein Antiinfektivum und ein keratolytisches Mittel. Die Zusammensetzungen kommen bei der Behandlung von Pilz-, Bakterien- oder Virusinfektionen zur Anwendung.

Die AT 11 910 U1 betrifft Zusammensetzung umfassend Chlorhexidin, Bisphosphonat, ein nicht steroidales entzündungshemmendes Arzneimittel (NSAID) und einen Immunomodulator (Tetrazyklin) unter anderem für die Behandlung oder Prävention oraler, mukosaler oder dermaler Infektionen oder Entzündungen.

Die DE 10 2008 034944 A1 betrifft Mikroemulsionen auf Honigbasis, die zusammen mit weiteren Wirkstoffen, under anderem auch NSAIDs, durch topische Anwendung in die Haut oder oral, nasal oder perkutan in den Körper eingebracht werden können.

Die WO 2011/060083 A1 betrifft Verfahren zur Vorbeugung oder Behandlung von Außenohrentzündungen mit Zusammensetzungen enthaltend Antibiotika, Antimykotika, Antiparasitika, antivirale Wirkstoffe, NSAIDs, Analgetika, Anästhetika und/oder Steroide.

Die WO 02/078648 A2 betrifft topische pharmazeutische Zusammensetzungen enthaltend ein Antimykotikum, z.B. Terbinafin, und einen weiteren Arzneistoff, z.B. Diclofenac oder Indomethacin.

Gegenstand der vorliegenden Erfindung sind Arzneimittel zur topischen Behandlung von bakteriellen Dysbiosen und manifesten Infektionskrankheiten, einschließlich von Mischinfektionen mit Pilzen und anderen Mikroorganismen. Demgemäß betrifft die vorliegende Erfindung eine Emulsion zur topischen Behandlung von dermalen Infektionen und Schleimhautinfektionen, insbesondere von urogenitalen Infektionskrankheiten, insbesondere zur Anwendung in der topischen Behandlung von urogenitalen bakteriellen Infektionen, die dadurch gekennzeichnet ist, dass ein antimikrobieller Wirkstoff, ausgewählt aus einem Antibiotikum und einem Antiseptikum, und ein NSAID kombiniert eingesetzt werden, wobei (a) das NSAID Diclofenac in einer Konzentration von 0,1 bis 0,5 Gewichtsprozent, Indometacin in einer Konzentration von 0,1 bis 0,4 Gewichtsprozent, Naproxen in einer Konzentration von 1 bis 5 Gewichtsprozent, Ibuprofen in einer Konzentration von 0,5 bis 2,5 Gewichtsprozent, Dexibuprofen in einer Konzentration von 0,25 bis 1,25 Gewichtsprozent, Ketoprofen in einer Konzentration von 0,25 bis 1,25 Gewichtsprozent, Mefenaminsäure in einer Konzentration von 0,5 bis 4 Gewichtsprozent, oder Lornixocam in einer Konzentration von 0,02 bis 0,04 Gewichtsprozent ist, wobei das NSAID in Salzform vorliegt; (b) das Gewichtsverhältnis der Wasser zur Ölphase in dieser Emulsion zwischen den Werten 2,0 und 2,7 liegt, wobei das Gewichtsverhältnis unter Einbeziehen der in den Phasen gelösten Stoffen berechnet wird, wobei Emulgatoren weder der Wasser- noch der Ölphase zugerechnet werden, und (c) der pH Wert der Emulsion nicht unter dem Wert 6,5 und nicht über 8,5 vorzugsweise im Bereich 7,0 bis 8 liegt.

Weiterhin betrifft die Erfindung die genannte Emulsion zur Anwendung in medizinischen Verfahren gemäß den unabhängigen Ansprüchen 6 und 11-13, sowie Verfahren zur Herstellung der genannten Emulsion gemäß den unabhängigen Ansprüchen 14 und 15.

Bakterielle Fehlbesiedelungen der Scheide bis hin zu symptomatischen Vaginalinfektionen sind häufig. Der Übergang von der asymptomatischen noch nicht entzündlichen Fehlbesiedelung zur symptomatischen Erkrankung wird durch die Begriffe Vaginose und Vaginitis näher bestimmt. Die Bezeichnungen aerobe Vaginose/Vaginitis und anaerobe Vaginose/Vaginitis (syn.: bakterielle Vaginose) stellen eine genauere Bestimmung der ursächlichen Erreger dar. Ein Problem, das allen vaginalen Infektionen, unabhängig vom Erregerorganismus gemeinsam ist, stellt die Ausbildung sogenannter Biofilme dar. Die Schleimhautoberflächen der betroffenen Organe (Vagina, Harnröhre, Blase, Penis) werden durch diese Biofilme, überzogen. In diesen sind die Mikroorganismen von einer Schicht aus Schleimsubstanzen umhüllt. Die einzelnen mikrobiellen Bestandteile des Biofilms übernehmen unterschiedliche Aufgaben im Zellverbund und tauschen auch Resistenzmechanismen aus. Die am häufigsten bei bakteriellen Dysvaginosen aufzufindenden Keime sind überdurchschnittlich starke Biofilmbildner. Dadurch wird der therapeutische Zugang bei einer manifesten Infektion massiv erschwert und Chronifizierung bzw. häufige Rückfälle im Infektionsgeschehen sind die Folge. Rezente Untersuchungen bestätigen, dass eine Wiederinfektion häufig durch den Partner erfolgt. Eine Partnerbehandlung ist daher immer angezeigt. Bei Männern finden sich die Keimreservoirs zumeist in der Umschlagfalte der Vorhaut und/oder im ersten Drittel der Urethra.

Bakterielle Dysbiosen und Infektionen lassen sich durch eine Arzneimittelkombination bestehend aus einem antimikrobiellen Arzneistoff mit einem antiadhäsiven Wirkstoff effizient therapieren. Durch die Adhäsionshemmung wird der Biofilm aufgebrochen und vom Epithel des Wirtes abgelöst. Damit werden gleichzeitig alle darin enthaltenen Keime freigesetzt und der direkten antimikrobiellen Behandlung wieder zugänglich gemacht. Eine besonders beanspruchte Arzneistoffklasse unter den antiadhäsiven Wirkstoffen sind die nicht-steroidalen anti-inflammatorischen Arzneistoffe (NSAIDs), Diese weisen zusätzlich zum antiadhäsiven Effekt eine entzündungshemmende Wirkung und eine sofortige Schmerzhemmung auf, was in Anbetracht der häufig mit Schmerzen verbundenen Vaginalinfektionen ein weiterer besonderer Vorteil ist. Da chronische Infektionen zugleich mit einer chronischen Entzündung einhergehen, ist auch die entzündungshemmende Wirkung vor allem bei chronischen Infektionen von besonderer Bedeutung. Entscheidend für das Aufbrechen des Biofilms ist die Anwesenheit des NSAID in den beschriebenen Konzentrationen und Bedingungen in einer Emulsion in der beschriebenen Zusammensetzung und in den beschriebenen Mengenverhältnissen. Im Sinne der Erfindung vorgesehene NSAIDs sind Diclofenac, Ibuprofen, Dexibuprofen, Ketoprofen, Lornoxicam, Mefenaminsäure, Indometacin oder Naproxen.

Die übliche Konzentration, in der Diclofenac dermal topisch eingesetzt wird, liegt im Bereich von 1 bis 2% (10 mg/bzw. 20 mg/g). Im Rahmen der vorliegenden Erfindung ist es bevorzugt, dass Diclofenac eingesetzt wird in einer Menge von 0,1% bis 0,5 % (1 - 5 mg pro g Creme), vorzugsweise von 0,2.%. bis 0,4%. Die übliche Konzentration, in der Ibuprofen topisch eingesetzt wird, liegt bei 5% (50 mg/g Creme/Gel). Im Rahmen der Erfindung wird Ibuprofen eingesetzt in einer bevorzugten Menge von 0,5 bis 2,5% (5 - 25 mg pro g Creme), vorzugsweise von 1 bis 2%. Weitere Beispiele für bevorzugte NSAIDs und deren bevorzugte Mengen ("Erfindungsgemäße Konzentration") können aus Tabelle A ersehen werden.

**Tab: A - Beispiele für übliche und bevorzugte erfindungsgemäße Konzentrationen**

| Wirkstoff | Einzeldosis | Tageshöchstdosis | Beispiel | Einzeldosis | Tageshöchstdosis | Beispiel | **Erfindungsgemäße Konzentration** |
|---|---|---|---|---|---|---|---|
| | oral/i.v. | | | top isch | | | |
| Diclofenac Natrium | 50 - 75 mg | 150 - 225 mg | Voltaren 50 mg Tabletten | | | Voltadol Schmerzgel 1%, 10 mg/g | **0,2** - **0,5 Gew%** |
| | | | 75 mg Amp. | 40 - 80 mg | 240 mg | 2%, 20 mg/g | |
| Indometacin | 25 - 75 mg | 50 - 150 mg | Indocid 25 mg | 20-40 mg | 40-80 mg | IndometGel | **0,1** - **0,4 Gew.%** |
| | | | 75 mg ret. | | | 1%, 10 mg/g | |
| Naproxen | 250 - 500 mg | 1000 mg | Naproxen FT 250/500 mg | | | | |
| | | | Naproxen Susp. 50 mg/ml | | | | **0,5 - 2,5 Gew%** |
| Ibuprofen | 200 - 800 mg | 2400 mg | Ibuprofen FT 200/ 400/800 mg | | | Ibutop Creme/ Gel | |
| | | | | 100 -250 mg | 1000 mg | 5%, 5g/100 g | **0,5-2,5 Gew.%** |

Im Rahmen der Erfindung besonders bevorzugt sind die folgenden NSAIDs:

| **NSAID** | **Bevorzugte Konzentration (Gew.%)** | **Bevorzugte Einzeldosis(mg)** |
|---|---|---|
| Diclofenac | 0,1 - 0,5 (vorzugsweise 0,2 - 0,4) | 4 bis 8 mg |
| Indometacin | 0,1 - 0,4 | 2 - 8 mg |
| Naproxen | 1 - 5 | 20 bis 100 mg |
| Ibuprofen | 0,5 - 2,5 | 10 bis 50 mg |
| Dexibuprofen | 0,25-1,25 | 5 bis 25 mg |
| Ketoprofen | 0,25-1,25 | 5 bis 25 mg |
| Mefenaminsäure | 0,5 - 4 | 10 - 40 mg |
| Lornixocam | 0,02 - 0,04 | 0,4 bis 0,8 mg |

Die Konzentrationseinschränkung It. Tabelle gilt für die Anwendung auf offenen Wunden (möglich im Fall dermaler Pilzinfektionen) und auf Schleimhäuten. Für die Anwendung auf weniger stark entzündeter Haut ist eine NSAID-Konzentration bis maximal zur üblichen topischen Menge möglich.

In einer bevorzugten Ausführungsform liegt die Emulsion in Form einer Salbe oder einer Creme vor. Vorzugsweise ist die Emulsion zur Behandlung von urogenitalen Infektionskrankheiten, insbesondere zur Anwendung in der topischen Behandlung von vaginalen Infektionen und von Blasenentzündungen der Frau sowie zur lokalen Partnerbehandlung (Glans penis, Anfangsdrittel der Urethra).

Insbesondere zeigen die erfindungsgemäßen Präparate neben der optimierten Pharmakokinetik durch den Angriff unmittelbar am Infektionsort auch eine optimale Pharmakodynamik. Damit wird nicht nur eine besonders gute Wirksamkeit des antimikrobiellen Arzneistoffes ermöglicht, sondern auch das Zusammenwirken mit dem NSAID signifikant verbessert, indem durch eine niedrige Konzentration an NSAID trotzdem eine ausreichende Wirksamkeit gesichert wird, ohne die dabei auftretenden Nebenwirkungen (Reizungen, Brennen, etc.) in Kauf nehmen zu müssen. In der Praxis führt dies dazu, dass zuvor aus diesen Nebenwirkungsgründen nicht einsetzbare Kombinationen aus antimikrobiellen Arzneistoffen und NSAID mit der Lehre der vorliegenden Erfindung den Patientinnen zugänglich gemacht werden können und diese Patientinnen nunmehr einer erfolgreichen Therapie zugeführt werden können. Erfindungsgemäß hat sich aber gezeigt, dass für eine optimale pharmakodynamische Wirkung die Konzentration des NSAID in der wässerigen Phase von entscheidender Bedeutung ist. Die Verfügbarmachung des eingebrachten NSAIDs wird durch das Zusammenwirken des Herstellungsprozesses, des Wasser/Öl-Verhältnisses und des pH-Wertes, durch welchen das überwiegende Vorliegen des NSAIDs in seiner Salzform gewährleistet wird, erreicht.

Halbfeste Emulsionen (Öl in Wasser oder Wasser in Öl), vorzugsweise in Form einer Creme, sind besonders gut geeignet, um die Wirkstoffe effizient und konzentriert an die mit Biofilm behafteten Oberflächen heranzubringen. Die Viskosität der Emulsionen wird stark durch das Wasser zu Öl Verhältnis bestimmt. Überraschender Weise zeigte sich, dass bei der Formulierung der Emulsion dem Verhältnis Wasser: Öl eine besondere Bedeutung zukommt. Bei höherem Anteil der Fettbestandteile wird die Entfaltung der Wirksamkeit behindert. Hingegen kommt es bei einem niedrigeren Fettanteil zu einer stärkeren Reizwirkung.

Es ist bekannt, dass die Findung einer Arzneiform zur topischen Applikation in der Scheide dadurch erschwert ist, dass es bei der Anwendung leicht zu einem Auslaufen des Arzneimittels kommen kann, was eine zuverlässige Dosierung erschwert. In Anbetracht des relativ engen therapeutischen Fensters setzen Medikamente gemäß der vorliegenden Erfindung zur Erzielung des gewünschten therapeutischen Effektes den sicheren Verbleib des Wirkstoffes, vor allem der NSAID-Komponente am Wirkort voraus. Deshalb bewegt sich im Arzneimittel das Verhältnis der Ölphase, welche das Antimykotikum enthält, zur Wasserphase, in welcher sich das NSAID befindet, in einem relativ engen Bereich. Wenn die Viskosität also in stärkerem Ausmaß durch Erhöhung des Anteils der wässrigen Phase weiter herabgesetzt wird, ist ein unkontrollierter Verlust durch Auslaufen aus der Scheide zu erwarten. Flüssige Emulsionen oder Gele mit hohem Wassergehalt bzw. mit niedriger Viskosität sind als Arzneiformen für die vaginale Anwendung gemäß der vorliegenden Erfindung auszuschließen.

Da chronische Infektionen der Scheide zugleich mit einer chronischen Entzündung des Scheidenepithels verbunden sind, kann in solchen Fällen eine Heilung nur erzielt werden, wenn der Verbleib des NSAID in der Scheide sichergestellt ist.

Zur Erzielung eines optimalen therapeutischen Effektes soll das Wasser: Öl Verhältnis den Wert von 2,7 nicht übersteigen. Über diesem Bereich wird der Wirkstoff zu schnell mit dem Scheidensekret ausgeschwemmt, wodurch keine ausreichende Zeit gegeben ist, um die zur Adhäsionsunterbindung beitragende Wirkung auf die äußere Schicht des Vaginalepithels, an welchem der Erreger haftet, zu entfalten. Der bei zu hohem Wasser: Öl Verhältnis sehr schnell ausgeschwemmte Wirkstoff kann allenfalls als Nebenwirkung zusätzlich auch einen reizenden Effekt verursachen.

In gleicher Weise, wie ein zu hoher Wasseranteil in der Emulsion einen negativen Effekt ausübt, ist auch ein zu hoher Fettanteil zu vermeiden. In Anbetracht der niedrigen Konzentration, in welcher das NSAID eingesetzt wird, ist es zur Erzielung des therapeutischen Effektes von besonderer Wichtigkeit, dass die Freisetzung des Wirkstoffes am Wirkort rasch und nicht protrahiert erfolgt. Bei einer langsamen Freisetzung, wie sie aus der öligen Phase erfolgt, ist nicht gewährleistet, dass die erforderliche therapeutische Konzentration am Wirkort erreicht wird. Aus diesem Grund soll das Wasser: Öl Gewichtsverhältnis in der Emulsion den Wert von 2,0 nicht unterschreiten. Somit ergibt sich auch für den Wert des Wasser-Öl Gewichtsverhältnisses der erfindungsgemäßen Emulsionen ein Fenster zwischen 2,0 und 2,7, vorzugsweise zwischen 2,1 und 2,6, noch mehr bevorzugt zwischen 2,2 und 2,5.

Es ist wesentlich, dass die NSAIDs gemäß der Erfindung in Salzform (bzw. ionischer Form) vorliegen und zwar sowohl bei der Einarbeitung als auch bei der Anwendung.

Deshalb ist deren Einbringung in die Formulierung von Bedeutung. Bei einer Einarbeitung des NSAID in seiner freien Form oder bei der Einarbeitung als Salz in die ölige Phase wird der therapeutische Effekt massiv beeinträchtigt. Während das Antimykotikum vorzugsweise in die ölige Phase eingearbeitet wird und in dieser vorliegt, wird daher nach dem Verfahren gemäß der Erfindung das NSAID vor Herstellung der Emulsion in der Regel in die wässrige Phase eingebracht. Alternativ dazu kann das feste Salz des NSAID in feinkristalliner oder mikronisierter Form, bzw. als Hydrogel in die (weitgehend) fertige Emulsion eingearbeitet werden.

Eine zügige Freisetzung ist dann gewährleistet, wenn in der Emulsion der Wirkstoff in der wässrigen Phase vorliegt, was voraussetzt, dass das NSAID als Salz vorliegt. Die meisten NSAIDs sind schwache Säuren mit einem pKa-Wert von 4 - 5 (Diclofenac 4,15, Ibuprofen 4,91, Mefenaminsäure 4,2, Indometacin 4,5, Naproxen 4,2). Demgemäß liegen sie bereits im schwach sauren Milieu zum Teil in freier Form vor und werden so in die Ölphase extrahiert, was zu einer reduzierten Wirkung oder zum Wirkungsverlust führen kann. Da mit sinkendem pH-Wert der Wirkstoffanteil des als freie Säure vorliegenden NSAID in der Ölphase zunimmt, ist ein pH-Wert von mindestens pH = 6 zweckmäßig, um bei den Medikamenten in den Wirkstoffkonzentrationen gemäß der Erfindung einen therapeutischen Effekt zu haben. Bei NSAIDs kann es aber zu einer Abnahme der chemischen Stabilität im basischen Milieu kommen kann (im Fall von Diclofenac ab pH 8,00 - 8,5). Ebenso muss beachtet werden, dass der pH-Wert einer Emulsion die grundsätzliche physiologische Verträglichkeit beeinflusst. Deshalb sind auch bezüglich des pH-Wertes der Formulierung relativ enge Grenzen gesetzt. Gemäß der Erfindung hat die (wässerige Phase der Emulsion) einen pH-Wert im Bereich von 6,5 bis 8,5, vorzugsweise von 7 - 8.

Im Rahmen der Erfindung werden Antibiotika oder Antiseptika als antimikrobielle Wirkstoffe eingesetzt.

Unter den bakteriellen Mikroorganismen, welche sich in den vaginalen Biofilmen finden, spielen typische meist fakultativ anaerobe Darmkeime, wie Enterobacter, E. coli, Klebsiella pneumoniae und Enterokokken, aber auch Ureaplasmen und Mykoplasmen, sowie Gardnerella vaginalis, Prevotella spp. und Mobiluncus eine besondere Rolle. Bevorzugte Antibiotika im Rahmen der vorliegenden Erfindung, die auch besonders zur Behandlung dieser Keime geeignet sind, sind Phosphomycin, Clindamycin, Metronidazol, Nitrofurantoin, Nitrofurazon, Nitrofurantoin, Nifuratel, Nifuroxacin, Nitroxolin, Trimethoprim, Sulfadiazin, Cotrimoxazol.

Antiseptika können sowohl auf nicht bakterielle Mikroorganismen wie Trichomonaden, als auch auf Bakterien wirken. Bei höheren Konzentrationen üben Antiseptika eine antibakterielle Wirkung aus und sind alleine oder gemeinsam mit einem Antibiotikum zur Kombination mit einem antiadhäsiven Wirkstoff gemäß der Erfindung geeignet. Die antimikrobielle Wirkung der Antiseptika beruht vorwiegend auf der Störung der Integrität der Plasmamembran. Wegen des im Prinzip gleichen Aufbaus der Membran der Endothelzellen, haben diese Wirkstoffe ein inhärentes Potential zur Entzündungserregung. Da dieses Potential durch NSAID unterbunden wird, bieten die erfindungsgemäßen Kombinationspräparate von Antiseptika einen ganz besonderen therapeutischen Vorteil.

Bevorzugte Antiseptika sind quartäre Ammoniumsalze, wie Benzalkoniumchlorid, und Dequaliniumchlorid, sowie Phenoxyethanol. Bevorzugte Konzentration sind mindestens 0,2 Gewichtsprozent für Benzalkoniumchlorid, mindestens 0,2 Gewichtsprozent für Dequaliniumchlorid, und mindestens 2 Gewichtsprozent für Phenoxyethanol. Im Zusammenhang mit der vorliegenden Erfindung sollen unter den Begriffen "antimikrobieller Wirkstoff", "antibakterieller Wirkstoff", "Antibiotika", "Antiseptika", etc. Substanzen verstanden werden, die im üblichen pharmazeutischen Gebrauch als solche Wirkstoffe angesehen werden. Welche Substanzen als solche Wirkstoffe angesehen werden, kann beispielsweise aus dem Arzneimittelverzeichnis Rote Liste (Rote Liste Service GmbH (Herausgeber und Verlag), Rote Liste 2014 - Arzneimittelverzeichnis für Deutschland (einschließlich EU-Zulassungen und bestimmter Medizinprodukte), 2047 Seiten, ISBN 978-3-939192-80-0) entnommen werden. Keine antibakteriellen Wirkstoffe im Sinne der Erfindung sind Benzylalkohol, Ethanol oder Propanol.

Die Zusammensetzung der erfindungsgemäßen Arzneistoffkombinationen ist in den beschriebenen Kombinationen für die Behandlung auch komplexer chronischer Scheidenentzündungen ganz besonders geeignet.

In speziellen Applikationen ist es zweckmäßig, der Emulsion einen Geruchsstoff hinzuzufügen. Der Zusatz eines Terpens zur Emulsion, vorzugsweise von Farnesol, welches zusätzlich einen Biofilm-hemmenden Effekt hat, stellt daher einen bevorzugten Gegenstand der Erfindung dar.

Die erfindungsgemäßen Emulsionen sind zur Anwendung auf Schleimhäuten, insbesondere von urogenitalen Infektionen geeignet. Ungeeignet für die vaginale Applikation sind beispielsweise Zusammensetzungen, die einen hohen Gehalt an schleimhautschädigenden Stoffen, beispielsweise Ethanol oder Isopropanol, aufweisen. Es ist daher bevorzugt, dass die erfindungsgemäße Emulsion nicht mehr als 10 % Ethanol enthält. Es ist außerdem bevorzugt, dass die erfindungsgemäße Emulsion nicht mehr als 20 % Isopropanol enthält. Da verhornte Schichten bei Schleimhäuten, z.B. im Urogenitalbereich, nicht vorkommen, ist ein keratoytischer Effekt nicht erwünscht. Aus diesem Grund ist die stark sauer wirkende, keratinolytische Salicylsäure kein erfindungsgemäßes NSAID.

Emulsionen gemäß der vorliegenden Erfindung sind auch zur Behandlung von urogenitalen Schleimhauterkrankungen des Mannes an der Glans penis und in der Urethra geeignet.

Blasenentzündungen sind weit verbreitet. Sie werden meist durch bakterielle Infektion verursacht. Die Blase ist für eine topische Behandlung schlecht zugänglich. Da sich allerdings die Harnröhre der Frau im vorderen Scheideneingang befindet, verläuft der häufigste Infektionsweg von der Scheide aus in die Harnröhre hinein. Außerdem werden in der urologischen und gynäkologischen Praxis auch häufig Beschwerdebilder gesehen, welche sich auf eine isolierte Entzündung der Harnröhre zurückführen lassen. Die Vagina wird somit zum primären Keimreservoir für Harnröhren- und Blasenentzündungen. Wenn auch eine Unterstützung der antibiotischen Behandlung von akuten Blasenentzündungen durch die orale Gabe von adhäsionshemmenden Pflanzenextrakten, welche sich an der Blasenwand festsetzen, erreicht werden kann, so ist dennoch die Sanierung der meist ebenso von der Infektion betroffenen Scheidenflora die Grundvoraussetzung für eine nachhaltige Heilung. Diese kann mit den erfindungsgemäßen Arzneimitteln erreicht werden. Aus diesem Grund sind Arzneimittel gemäß der vorliegenden Erfindung effiziente Therapeutika gegen Blasenentzündung, sowohl alleine, als auch in Kombination mit nur in der Blase wirkenden Arzneimitteln.

Abgesehen von rein bakteriellen Infektionen kommt es häufig auch zu Mischinfektionen mit Pilzen (überwiegend Hefen der Gattung Candida). In der WO 2007/131253 A2 werden Arzneimittelkombinationen zur topischen Behandlung von Pilzinfektionen beansprucht, deren besondere Wirkung darauf beruht, dass zusätzlich zum antimykotischen Wirkstoff ein weiterer Wirkstoff hinzukommt, der die Adhäsion des Pilzes am Epithel unterbindet. Manche der genannten Mischinfektionen können durch die in der WO 2007/131253 A2 beanspruchten Kombinationen therapiert werden. In schweren Fällen von Mischinfektionen ist es allerdings zweckmäßig bzw. notwendig, sich nicht auf die Behandlung der Pilzinfektion zu beschränken, sondern das Arzneimittel durch einen antibakteriellen Wirkstoff in einer Tripelkombination zu ergänzen.

Der antimykotische Wirkstoff ist vorzugsweise ein Arzneistoff aus der Gruppe Nystatin, Ciclopirox oder Ciclopiroxolamin, oder einer aus der Gruppe der Azole (Imidazole, Triazole, Tetraazole) wie Clotrimazol, Fluconazol, Miconazol, Itraconazol, Tioconazol, Voriconazol, Bifonazol, Econazol, Isoconazol, Fenticonazol, Sertaconazol, Ketoconazol, Posaconazol, Quilseconazol, Oteseconazol (VT-1161), Ibrexafungerp (SCY-078).

Arzneimittel gemäß der vorliegenden Erfindung wurden vor allem zur Therapie von Vaginosen und Blasenentzündungen entwickelt. Die Erfindung betrifft daher die Anwendung der erfindungsgemäßen Emulsionen in der topischen Behandlung von Infektionskrankheiten, insbesondere zur Anwendung in der topischen Behandlung von urogenitalen Infektionen. Vorzugsweise ist die Infektionskrankheit eine mikrobielle (insbesondere eine bakterielle) urogenitale Infektion, insbesondere eine mikrobielle (insbesondere eine bakterielle) urogenitale Infektion der Frau. In einer besonders bevorzugten Ausführungsform ist die Infektionskrankheit eine vaginale Mischinfektionen durch Candida albicans und Bakterien wie Enterobacter, E.coli, Klebsiella pneumoniae, Gardnerella vaginalis, Prevotella spp. In einer weiteren bevorzugten Ausführungsform ist die Infektionskrankheit eine asymptomatische oder symptomatische bakterielle Vaginose oder eine asymptomatische oder symptomatische Dysbiose der Glans penis und/oder der männlichen Urethra.

Sowohl die Akne als auch der genetisch bedingte Haarausfall werden mit dem männlichen Sexualhormon Testosteron in Zusammenhang gebracht. Tatsächlich besteht der Beitrag dieses Hormons am pathogenetischen Geschehen primär in der Aktivierung von Talgdrüsen in der Haut und in den Haarbälgen. Der angesammelte Talg ist in der Folge ein ideales Substrat sowohl für eine bakterielle Infektion, vor allem mit Propionibakterien oder mit den Pilzen Candida und Malassezia. Während Malassezia vorwiegend mit der Pityriasis versicolor in Zusammenhang gebracht wird, steht bei der Akne Propionibacterium acnis im Vordergrund. Beim Haarausfall sind beide beteiligt. Die große Menge an Talg, welche sich in den Haarfollikeln ansammelt, ist ein ideales Substrat für die Ausbildung eines Biofilms. Grundsätzlich gelten für diesen Biofilm dieselben Kriterien wie für Biofilme, welche sich in der Scheide bilden. Auch in diesem Fall muss zunächst der Biofilm aufgebrochen und seine Anhaftung am Epithel aufgelöst werden, damit in der Folge das Antiinfektivum seine Wirkung ausüben kann. Demgemäß sind die Emulsionen gemäß der vorliegenden Erfindung auch zur Behandlung von Hautkrankheiten, vor allem von Pityriasis versicolor, Akne und Haarausfall sehr gut geeignet. Da die dermalen Anwendungen ein wesentlich größeres und deutlich weniger fest umschriebenes Areal betreffen können, als Scheideninfektionen, können in diesen Fällen spezielle Emulsionen bevorzugt zur Anwendung kommen. Insbesondere sind zur Behandlung des Haarausfalls Shampoos und zur Behandlung der Akne Akne-Cremes, Akne-Sticks oder Akne-Lösungen geeignet.

Die Erfindung wird anhand der nachfolgenden Beispiele näher erläutert, auf die sie jedoch nicht eingeschränkt ist.

### Beispiele:

### Herstellung von Basisrezeptur A, allgemeine Herstellungsvorschrift 1:

Die Komponenten Sorbitanmonostearat, Polysorbat 60, Cetylpalmitat, 2-Octyldodecanol und Cetostearylalkohol werden bei einer Temperatur von 70-75°C aufgeschmolzen. In die Klarschmelze werden unter Rühren bei einer Temperatur von 60 °C - 70 °C Clindamycin (und gegebenenfalls Clotrimazol) und anschließend Phenoxyethanol zugegeben. Parallel hierzu wird Diclofenac Natrium in gereinigtem Wasser unter Erwärmung aufgelöst. Die wässrige Lösung wird unter Rühren zur Ölphase zugegeben und homogenisiert. Unter langsamem Abkühlen unter weiterer Homogenisierung der entstehenden w/o-Emulsion findet eine Phasenumkehr statt, bei welcher eine hydrophile, homogene Creme entsteht.

| Bestandteile der Emulsion | | | |
|---|---|---|---|
| Clindamycin | | 2,00 | 2,00 |
| Diclofenac Na | | 0,20 | 0,30 |
| Sorbitanmonostearat | | 2,00 | 2,00 |
| Polysorbat 60 | | 1,50 | 1,50 |
| Cetylpalmitat | | 3,00 | 3,00 |
| 2-Octyldodecanol | | 13,50 | 13,50 |
| Cetostearylalkohol | | 10,00 | 10,00 |
| Phenoxyethanol | | 1,00 | 1,00 |
| Gereinigtes Wasser Ph.Eu. | | 66,80 | 66,70 |
| | | 100,00 | 100,00 |

### Emulsionen welche Phenoxyethanol als antibakterielles Agens enthalten

**Tab.: F1 - Phenoxyethanol als antibakterielles Agens**

| Zusammensetzung der Emulsion | | Gew% |
|---|---|---|
| Clotrimazol | | 1 |
| Diclofenac-Na | | 0,4 |
| Sorbitanmonostearat | | 2 |
| Polysorbat 60 | | 1,5 |
| Cetylpalmitat | | 3 |
| 2-Octyldodecanol | | 13,5 |
| Cetystearylalkohol | | 10 |
| Phenoxyethanol | | 4 |
| Gereinigtes Wasser Ph.Eu. | | 64,6 |
| Gesamt | | 100 |

**Fallbeispiel:** 24jährige Patientin (EH), seit 3 Jahren fast ständige Beschwerden durch Pilzinfektionen, im GV seit Jahren kaum möglich. Seit 1 Woche extreme Beschwerden. Brennen und Jucken im Introitus.

Gynäkologische Untersuchung (Gyn.U.): Vulva und Vaginalschleimhaut massiv gerötet, massiv rahmig-grünlicher Scheideninhalt, Sekretabstrich nativ: massenhaft an den Epithelzellen und an massenhaft Pilzhyphen haftende Leukozyten, Mischflora, wenig Lactobacillen, schmutziger Hintergrund - lytische Zellen, Intermediärflora.
Therapie: F1 über 2 Wochen, 0-0-1 Hb vaginal appliziert, anschließend F1 bei Bedarf. Ko. nach 3 Jahren: seit letzter Therapie beschwerdefrei;
Gyn.U.: Schleimhaut bland, normale Scheidenflora, Lactobacillenflora.

### Beispiel: Emulsionen, welche Dequaliniumchlorid enthalten

**Tab: F2 - Dequaliniumchlorid als antibakterielles Agens**

| Zusammensetzung der Emulsion | | Gew% |
|---|---|---|
| Clotrimazol | | 1 |
| Diclofenac Na | | 0,3 |
| Sorbitanmonostearat | | 2 |
| Polysorbat 60 | | 1,5 |
| Cetylpalmitat | | 3 |
| 2-Octyldodecanol | | 13,5 |
| Cetostearylalkohol | | 10 |
| Phenoxyethanol | | 1 |
| Dequaliniumchlorid | | 0,4 |
| Gereinigtes Wasser Ph.Eu. | | 67,3 |
| Gesamt | | 100 |

**Fallbeispiel:** 40jährige Patientin (AKS), seit Jahren fast monatlich Beschwerden durch Pilzinfektionen, jeweils über ca. 1 Woche bis 10 Tage, GV seit Jahren kaum möglich. Seit 1 Woche zusätzlich Vaginalsekret dünnflüssig und übelriechend. Brennen und Jucken im Introitus.

Gynäkologische Untersuchung: Schleimhaut stark gerötet, Sekret dünnflüssig, leicht grünlich. Sekretabstrich nativ: reichlich Biofilmplaques auf dicken Pilzhyphen, Leukozyten +++, kaum Lactobacillen, Intermediärflora.
Therapie: F2 über 1 Woche, 0-0-1 Hb vaginal appliziert.
Ko. nach 2 Jahren: seit 2 Jahren beschwerdefrei, nur gelegentlich sehr leichte Pilzinfektion, mit restl. F2 behandelt;
Gyn.U.: Schleimhaut bland, Lactobacillenflora

**Tab.: F3 - Dequaliniumchlorid als antibakterielles Agens**

| Zusammensetzung der Emulsion | | Gew% |
|---|---|---|
| **Clotrimazol** | | 1,00 |
| **Diclofenac Na** | | 0,20 |
| Sorbitanmonostearat | | 2,00 |
| Polysorbat 60 | | 1,50 |
| Cetylpalmitat | | 3,00 |
| 2-Octyldodecanol | | 13,50 |
| Cetostearylalkohol | | 10,00 |
| Propylenglykol | | 7,00 |
| **Dequaliniumchlorid** | | 0,40 |
| Gereinigtes Wasser Ph.Eu. | | 61,40 |
| Gesamt | | 100,00 |

**Fallbeispiel:** 31jährige Patientin (FJ), seit ca. 3 Jahren fast monatlich prämenstruelle Beschwerden, jeweils über ca. 1 Woche bis 10 Tage, durch rezidivierende Pilzinfektionen. Seit 1 Woche zusätzlich Vaginalsekret dünnflüssig und übelriechend. Brennen und Jucken im Introitus.

Gyn.U: Schleimhaut leicht gerötet, Sekret dünnflüssig, Odor. Sekretabstrich nativ: bakterielle Vaginose (RG III), zusätzlich Pilzhyphen, reichlich Leukozyten.

Therapie: F3 über 1 Woche, 0-0-1 Hb vaginal appliziert.

Kontrolluntersuchung nach 4 Monaten: subjektiv seit Therapie keine Beschwerden mehr. Gyn.U: Schleimhaut bland, Sekretabstrich nativ: normale Scheidenflora (RG I)

### Beispiel: Emulsionen, welche Clindamycin enthalten

**Tab: F4**

| Zusammensetzung der Emulsion | Gew% |
|---|---|
| **Clindamycin** | **2** |
| **Diclofenac Na** | **0,3** |
| Sorbitanmonostearat | 2 |
| Polysorbat 60 | 1,5 |
| Cetylpalmitat | 3 |
| 2-Octyldodecanol | 13,5 |
| Cetostearylalkohol | 10 |
| Propylenglykol | 7 |
| Phenoxyethanol | 1 |
| Gereinigtes Wasser Ph.Eu. | 59,7 |
| Gesamt | 100 |

**Fallbeispiel:** 31jährige Patientin (EK), seit Monaten therapieresistente. bakterielle Vaginose, übelriechender Ausfluss, V.a. postmenstruell. Im mikrobiol Abstrichbefund reichlich Gardnerella und Prevotella.

Gyn.U: Schleimhaut stark gerötet, Vaginalsekret dünnflüssig und übelriechend. Sekretabstrich nativ: reichl. Clue cells, Leukozyten +++, bakterielle Vaginose (RG III) Therapie: F4 über 1 Woche, 0-0-1 vaginal appliziert., anschließend Hylaktiv Vagilact. Kontrolle nach 2 Jahren: seit o.g.Therapie beschwerdefrei, einmal sehr leichte Pilzinfektion; Gyn.U.: Schleimhaut bland, normale Lactobacillenflora (RG I)

### Beispiel: Emulsionen, welche Phosphomycin-Trometamol enthalten

**Tab: F5**

| Zusammensetzung der Emulsion | Gew% |
|---|---|
| **Phosphomycin-Trometamol** | 2 |
| **Diclofenac-Na** | 0,4 |
| Sorbitanmonostearat | 2 |
| Polysorbat 60 | 1,5 |
| Cetylpalmitat | 3 |
| 2-Octyldodecanol | 13,5 |
| Cetystearylalkohol | 10 |
| Phenoxyethanol | 1 |
| Gereinigtes Wasser Ph.Eur. | 66,6 |
| Total | 100 |

**Fallbeispiel:** 69jährige Patientin (ET), seit ca. 2 Jahren rezidivierende Harnwegsinfekte, immer wieder mit Ciproxin behandelt. Seit 2 Wochen wieder verstärkter Harndrang und Brennen im Introitus.

Gyn.U: Druckschmerz über Blase; Schleimhaut atroph, blutet auf Kontakt, Muttermund atroph, verlötet, mit Cervixbrush eröffnet, Sekret: Atrophie, aerobe Mischflora (RGIII), reichlich Leukozyten.

Therapie: F5 über 1 Woche, 0-0-1 Hb vaginal appliziert, zusätzlich Ovestin.

Kontrolluntersuchung nach 2 Wochen: subj. seit Therapie keine Beschwerden mehr.

Gyn.U: Schleimhaut bland, kein DS über Blase und Urethra; Sekretabstrich nativ: beginnend normale Scheidenflora (RG I), keine Leukozyten.

**Tab.: F6**

| Zusammensetzung der Emulsion | Gew% |
|---|---|
| **Phosphomycin-Trometamol** | 2 |
| **Clotrimazol** | 1 |
| **Diclofenac Na** | 0,3 |
| Sorbitanmonostearat | 2 |
| Polysorbat 60 | 1,5 |
| Cetylpalmitat | 3 |
| 2-Octyldodecanol | 13,5 |
| Cetostearylalkohol | 10 |
| Propylenglykol | 7 |
| Phenoxyethanol | 1 |
| Gereinigtes Wasser Ph.Eu. | 58,7 |
| Gesamt | 100 |

**Fallbeispiel:** 36jährige Patientin (CS), vor 2 Jahren erstmals HWI nach Thailandurlaub mit Makrohämaturie und lang andauerndem Brennen, seit 2 Monaten wieder UB-Schmerzen und HWI (wieder nach Thailandurlaub), immer wieder Brennen v.a. nach dem Urinieren, seit 2 Wochen wieder verstärkter Harndrang, nach AB-Behandlung (1 Woche) keine Besserung der Blasenbeschwerden aber nun zusätzlich Jucken und Brennen im Introitus.

Gynäkologische Untersuchung: Druckschmerz über Blase; Vaginal-Schleimhaut vom Aspekt her eher unauffällig, ausgeprägte Cervicitis mit ca. 1 cm messender blutiger Erosion um den Muttermund, Sekret/ Nativpräparat: bakterielle Vaginose, massenhaft Leukozyten, diese mit Bakterien belegt, vereinzelt Lactobacillen, Hyphen ++. Mikrobiol Abstrich - Sekretkultur: massenhaft E. coli.

Therapie: F6 über 10 Tage, 0-0-1 vaginal appliziert, zusätzlich 2 Btl Monuril im Abstand von 3 Tagen.

Kontrolluntersuchung nach 4 Wochen: subj. seit Therapie keine Beschwerden mehr. Gyn.U: Schleimhaut bland, kein Druckschmerz über Blase und Urethra; Sekretabstrich nativ: normale Scheidenflora (RG I), keine Leukozyten.

**Tab: F7**

| Zusammensetzung der Emulsion | Gew% |
|---|---|
| **Phosphomycin-Trometamol** | **2** |
| **Diclofenac Na** | **0,3** |
| Sorbitanmonostearat | 2 |
| Polysorbat 60 | 1,5 |
| Cetylpalmitat | 3 |
| 2-Octyldodecanol | 13,5 |
| Cetostearylalkohol | 10 |
| **Propylenglykol** | **7** |
| Phenoxyethanol | 1 |
| Gereinigtes Wasser Ph.Eu. | 59,7 |
| Gesamt | 100 |

**Beispiel:** Einfluss des Gewichtsverhältnisses wässerige Phase/Öl auf die klinische Wirksamkeit des NSAIDs

Veränderungen der Viskosität zeigen überraschenderweise schon bei geringer Variationsbreite deutliche Einflüsse auf die klinische Wirksamkeit. Die Herstellung der genannten Beispiele erfolgt nach allgemeiner Arbeitsvorschrift 1 durch Variationen im Gehalt der fettigen Bestandteile und des Wassergehaltes.

Eine Erhöhung des Wasseranteils und damit Erniedrigung der Viskosität führt über eine verstärkte Freisetzung und stärkere Benetzung der Schleimhäute zu lokalen Reizerscheinungen und verminderter klinischer Wirksamkeit.

**Tab.: Einfluss des Gewichtsverhältnisses wässerige Phase/Öl auf die klinische**

| | **Phase** | **Fettanteil / Viskosität erniedrigt** | | **Basisformulierungen** | | **Fettanteil / Viskosität erhöht** | |
|---|---|---|---|---|---|---|---|
| Clotrimazol | Öl | 1 | 1,0 | 1,0 | 1,0 | 1,0 | 1,0 |
| Diclofenac-Na | Wasser | 0,3 | 0,3 | 0,3 | 0,3 | 0,3 | 0,3 |
| Sorbitanmonostearat | - | 2,0 | 2,0 | 2,0 | 2,0 | 2,0 | 2,0 |
| Polysorbat 60 | - | 1,5 | 1,5 | 1,5 | 1,5 | 1,5 | 1,5 |
| Cetylpalmitat | Öl | 3,0 | 3,0 | 3,0 | 3,0 | 3,0 | 3,0 |
| 2-Octyldodecanol | Öl | 13,5 | 13,5 | 13,5 | 13,5 | 13,5 | 14,5 |
| Cetystearylalkohol | Öl | 7,5 | 5 | 10 | 10 | 14 | 16 |
| Benzylalkohol | Öl | 1,0 | 1,0 | 1,0 | | 1,0 | 1,0 |
| Phenoxyethanol | Öl | | | | 1,0 | | |
| Propylenglykol | Wasser | | | | 7 | | |
| Wasser | Wasser | 70,2 | 72,7 | 67,75 | 60,7 | 63,7 | 60,7 |
| Total | | 100 | 100 | 100 | 100 | 100 | 100 |
| Klinische Wirksamkeit | | **reizend** | **reizend** | **entspricht** | **entspricht** | **erniedrigt** | **erniedrigt** |
| | | | | | | | |
| Wasserphase gesamt | | 70,5 | 73,0 | 68,0 | 68,0 | 64,0 | 61,0 |
| Fettphase gesamt | | 26 | 23,5 | 28,5 | 28,5 | 32,5 | 35,5 |
| **wässrige Phase / Fet**t | | **2,7** | **3,1** | **2,4** | **2,4** | **2,0** | **1,7** |

### Wirksamkeit

Formulierungen mit einem Verhältnis von wässriger Phase zur Fettphase außerhalb 2,0 bis 2,7 sind Referenzformulierungen.

Zur Berechnung des Gewichtsverhältnisses der Wasser- zur Ölphase werden die einzelnen Anteile der Wasser- und der Ölphase wie in der Tabelle gezeigt aufsummiert. Da Emulgatoren, z.B. Sorbitanmonostearat und Polysorbat 60, an den Grenzflächen zwischen den zwei Phasen liegen, sind sie weder der Wasser- noch der Ölphase zuzurechnen.

Berechnet man das Verhältnis der wässrigen Phase zur Ölphase der konzentrierten Emulsion (d.h. dem Zwischenprodukt aus Komponenten A, B, J, C, E, G, H und der Hälfte von K) von Beispiel 2 der WO 02/0768648 A2, erhält man ein Verhältnis von 3,1 (Ölphase: Terbinafin, Butylhydroxytoluol, Benzylalkohol, Isopropylmyristat, gesamt 11,52 g / 100 g; Wasserphase: Diclofenac-Natrium und Wassers, gesamt 35,94 g / 100 g; Verhältnis 3,1). Eine derartige Emulsion hat daher ein Wasser:Öl Verhältnis außerhalb des erfindungsgemäß bevorzugten Bereichs und wäre daher im Zusammenhang mit der vorliegenden Erfindung nicht erfindungsgemäß.

Alternativ könnte das Gewichtsverhältnis der Wasser- zur Ölphase ohne Einbeziehen der in den Phasen gelösten Stoffen (Clotrimazol, Diclofenac-Na, Benzylalkohol, Cetylstearylalkohol) berechnet werden. Bei dieser Berechnungsweise würde in Tab. 3 lediglich Wasser und Propylenglykol der Wasserphase, und Cetylpalmitat, 2-Octyldodecanol und Cetystearylalkohol der Ölphase zugerechnet werden. Die in Tab. 3 angegebenen Wasser-Öl Verhältnisse 2,7, 3,1, 2,4, 2,4, 2,0, 1,7 würden gemäß dieser Berechnungsweise den Werten 2,9, 3,4, 2,6, 2,6, 2,1, 1,8 entsprechen. Der erfindungsgemäße Bereich von 2,0 bis 2,7 würde bei dieser Berechnungsweise einem Bereich von 2,1 bis 2,9 entsprechen.

Im Rahmen der vorliegenden Erfindung soll die Berechnung des Gewichtsverhältnisses der Wasser- zur Ölphase aber wie in Tab. 3 gezeigt erfolgen, d.h. unter Einbeziehen der in den Phasen gelösten Stoffen.

Weitere Beispiele für den Einfluss des Gewichtsverhältnisses wässerige Phase/Öl auf die klinische Wirksamkeit:

| | | **Fettanteil/Viskosität erniedrigt** | | **Basisformulierungen** | | | **Fettanteil/Viskosität erhöht** | |
|---|---|---|---|---|---|---|---|---|
| Clotrimazol | Öl | 1,00 | 1,00 | 1 | 1 | 1 | 1,00 | 1,00 |
| Diclofenac- Na | Wasser | 0,30 | 0,30 | 0,25 | 0,3 | 0,4 | 0,30 | 0,30 |
| Sorbitanmonostearat | | 2,00 | 2,00 | 2 | 2 | 2 | 2,00 | 2,00 |
| Polysorbat 60 | | 1,50 | 1,50 | 1,5 | 1,5 | 1,5 | 1,50 | 1,50 |
| Cetylpalmitat | Öl | 3,00 | 3,00 | 3 | 3 | 3 | 3,00 | 3,00 |
| 2-Octyldodecanol | Öl | 13,50 | 13,50 | 13,5 | 13,5 | 13,5 | 13,50 | 14,50 |
| Cetystearylalkohol | Öl | 7,50 | 5,00 | 10 | 10 | 10 | 14,00 | 16,00 |
| Benzylalkohol | Öl | 1,00 | 1,00 | 1 | | | 1,00 | 1,00 |
| Phenoxyethanol | Öl | | | | 1 | 4 | | |
| Propylenglykol | Wasser | | | | 7 | | | |
| Gereinigtes Wasser Ph.Eur | Wasser | 70,20 | 72,70 | 67,75 | 60,7 | 64,6 | 63,70 | 60,70 |
| Total | | 100,00 | 100,00 | 100,00 | 100,00 | 100,00 | 100,00 | 100,00 |
| Klin. Wirksamkeit | | reizend | reizend | entspricht | entspricht | entspricht | erniedrigt | erniedrigt |
| wässrige Phase/Fet t | | **2,7** | **3,1** | **2,4** | **2,4** | **2,1** | **2,0** | **1,7** |
| Wasserphase | | 70,50 | 73,00 | 68,00 | 68,00 | 65,00 | 64,00 | 61,00 |
| Fet phase | | 26,00 | 23,50 | 28,50 | 28,50 | 31,50 | 32,50 | 35,50 |

### Beispiel: Einfluss des pH-Wertes auf die klinische Wirksamkeit des NSAIDs

Anhand von Emulsionen mit Clotrimazol und NSAIDs wurde der Einfluss des pH-Werts auf die klinische Wirksamkeit des NSAIDs untersucht. Es wurden Emulsionen mit verschiedenen Konzentrationen von Diclofenac Na hergestellt und auf ihre klinische Wirksamkeit untersucht.

**Tab.: Abhängigkeit der klinischen Wirksamkeit von der Konzentration des NSAIDs**

| | **Konz. Gew%** | **Konz. Gew%** | **Konz. Gew%** | **Konz. Gew%** |
|---|---|---|---|---|
| Clotrimazol | 1 | 1 | 1 | 1 |
| Diclofenac-Na | 0,1 | 0,25 | 0,5 | 0,75 |
| Sorbitanmonostearat | 2 | 2 | 2 | 2 |
| Polysorbat 60 | 1,5 | 1,5 | 1,5 | 1,5 |
| Cetylpalmitat | 3 | 3 | 3 | 3 |
| 2-Octyldodecanol | 13,5 | 13,5 | 13,5 | 13,5 |
| Cetystearylalkohol | 10 | 10 | 10 | 10 |
| Benzylalkohol | 1 | 1 | 1 | 1 |
| Gereinigtes Wasser Ph.Eur. | 67,9 | 67,75 | 67,5 | 67,25 |
| Total | 100 | 100 | 100 | 100 |
| **Ph** | **7,6** | **7,8** | **8,1** | **8,1** |
| Klin. Wirksamkeit | leicht erniedrigt | entspricht | entspricht, leicht reizend | schleimhautreizend |

Durch den gemeinsamen Einsatz von Clotrimazol und NSAIDs verändern sich durch die pH-Verschiebungen im System der Emulsion sowohl mikrobiologische als auch die chemische Stabilität [Lit. Pharmakopöe] gegenüber einer vergleichbaren Clotrimazolformulierung.

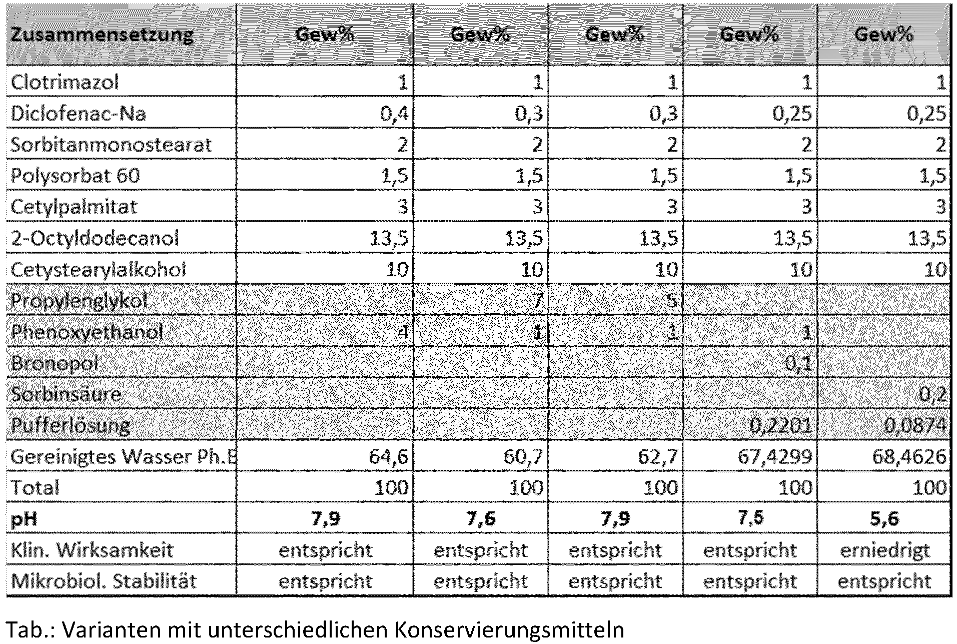

Die Variante mit einem pH-Wert von 5,6 ist eine Referenzformulierung.

### Beispiel: Shampoo zur Behandlung von pilzbedingtem Haarausfall:

**Tab: F8**

| Zusammensetzung der Emulsion | Gew% |
|---|---|
| Ketoconazol | 2 |
| Diclofenac Na | 0,3 |
| Ammoniumlaurylsulfat | 15,00 |
| Lauramid | 4,00 |
| Natriumchlorid | 1,00 |
| Farnesol | 1 |
| Dinatrium EDTA | 0,2 |
| Methylparaben | 0,08 |
| Propylparaben | 0,05 |
| Gereinigtes Wasser Ph.Eu. | 76,37 |
| | 100 |

Strukturformel von Ibrexafungerp (SCY-078).

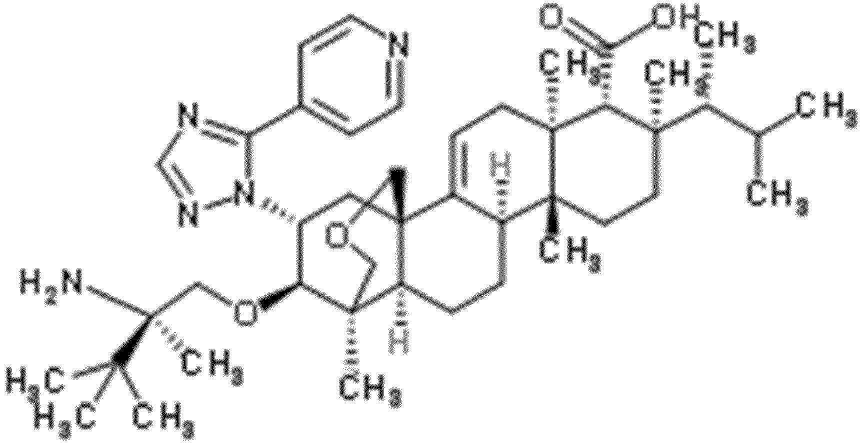

Strukturformel von Oteseconazol (VT-1161).

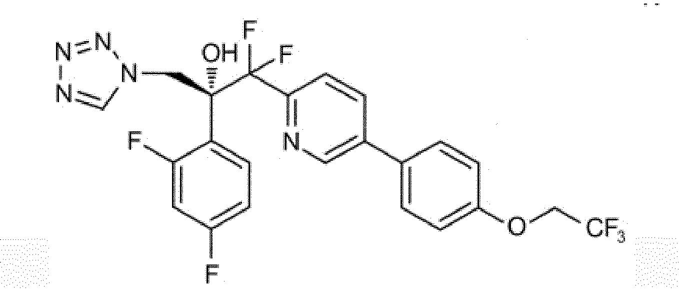

## Patentansprüche

1. Eine Emulsion zur topischen Behandlung von dermalen Infektionen und Schleimhautinfektionen, insbesondere von urogenitalen Infektionskrankheiten, **dadurch gekennzeichnet, dass** ein antimikrobieller Wirkstoff, ausgewählt aus einem Antibiotikum und einem Antiseptikum, und ein NSAID kombiniert eingesetzt werden, wobei (a) das NSAID Diclofenac in einer Konzentration von 0,1 bis 0,5 Gewichtsprozent, Indometacin in einer Konzentration von 0,1 bis 0,4 Gewichtsprozent, Naproxen in einer Konzentration von 1 bis 5 Gewichtsprozent, Ibuprofen in einer Konzentration von 0,5 bis 2,5 Gewichtsprozent, Dexibuprofen in einer Konzentration von 0,25 bis 1,25 Gewichtsprozent, Ketoprofen in einer Konzentration von 0,25 bis 1,25 Gewichtsprozent, Mefenaminsäure in einer Konzentration von 0,5 bis 4 Gewichtsprozent, oder Lornixocam in einer Konzentration von 0,02 bis 0,04 Gewichtsprozent ist, wobei das NSAID in Salzform vorliegt; (b) das Gewichtsverhältnis der Wasser zur Ölphase in dieser Emulsion zwischen den Werten 2,0 und 2,7 liegt, wobei das Gewichtsverhältnis unter Einbeziehen der in den Phasen gelösten Stoffen berechnet wird, wobei Emulgatoren weder der Wasser- noch der Ölphase zugerechnet werden, und (c) der pH Wert der Emulsion nicht unter dem Wert 6,5 und nicht über 8,5 vorzugsweise im Bereich 7,0 bis 8 liegt.

2. Eine Emulsion gemäß Anspruch 1, welche zusätzlich einen antimykotischen Wirkstoff enthält.

3. Eine Emulsion gemäß Anspruch 1 oder 2, in Form einer Salbe, einer Creme, eines Shampoos oder eines Sticks, vorzugsweise zur topischen Behandlung von dermalen Infektionen, vorzugsweise weiters enthaltend einen Geruchsstoff, vorzugsweise ein Terpen, insbesondere Farnesol.

4. Eine Emulsion gemäß einer der Ansprüche 1 bis 3, **dadurch gekennzeichnet, dass** der antimikrobielle Wirkstoff ein Antibiotikum ist, vorzugsweise Phosphomycin, Clindamycin, Metronidazol, Nitrofurantoin, Nitrofurazon, Nitrofurantoin, Nifuratel, Nifuroxacin, Nitroxolin, Trimethoprim, Sulfadiazin, oder Cotrimoxazol; oder dass der antimikrobielle Wirkstoff ein Antiseptikum ist, vorzugsweise ausgewählt aus der Gruppe bestehend aus: Benzalkoniumchlorid, vorzugsweise in einer Konzentration von mindestens 0,2 Gewichtsprozent; Dequaliniumchlorid, vorzugsweise in einer Konzentration von mindestens 0,2 Gewichtsprozent; und Phenoxyethanol, vorzugsweise in einer Konzentration von mindestens 2 Gewichtsprozent.

5. Eine Emulsion gemäß einer der Ansprüche 2 bis 4, **dadurch gekennzeichnet, dass** der antimykotische Wirkstoff Nystatin, Ciclopirox oder Ciclopiroxolamin, oder ein Antimykotikum aus der Gruppe der Azole, vorzugsweise Clotrimazol, Fluconazol, Miconazol, Itraconazol, Tioconazol, Voriconazol, Bifonazol, Econazol, Isoconazol, Fenticonazol, Sertaconazol, Ketoconazol, Posaconazol, Quilseconazol, Oteseconazol (VT-1161) oder Ibrexafungerp (SCY-078), ist.

6. Eine Emulsion gemäß einer der Ansprüche 1 bis 5 zur Anwendung in der topischen Behandlung von Infektionskrankheiten, insbesondere zur Anwendung in der topischen Behandlung von dermalen und urogenitalen Infektionskrankheiten, vorzugsweise wobei die Infektionskrankheit eine chronische Infektionskrankheit ist.

7. Emulsion zur Anwendung gemäß Anspruch 6, **dadurch gekennzeichnet, dass** die Infektionskrankheit eine Schleimhautinfektion, insbesondere eine urogenitale Infektion, bevorzugt eine mikrobielle urogenitale Infektion, insbesondere eine mikrobielle urogenitale Infektion der Frau, ist, vorzugsweise wobei die mikrobielle urogenitale Infektion eine bakterielle urogenitale Infektion ist.

8. Emulsion zur Anwendung gemäß Anspruch 6 oder 7, **dadurch gekennzeichnet, dass** die Infektionskrankheit eine vaginale Mischinfektion durch Candida albicans und Bakterien wie Enterobacter, E.coli, Klebsiella pneumoniae, Gardnerella vaginalis, Prevotella spp. ist.

9. Emulsion zur Anwendung gemäß einer der Ansprüche 6 bis 8, **dadurch gekennzeichnet, dass** die Infektionskrankheit eine asymptomatische oder symptomatische bakterielle Vaginose oder eine asymptomatische oder symptomatische Dysbiose der Glans penis und/oder der männlichen Urethra ist.

10. Emulsion zur Anwendung gemäß Anspruch 9, **dadurch gekennzeichnet, dass** die Infektionskrankheit eine dermale Pilzinfektion, vorzugsweise ausgewählt von Candidamykosen und Malasseziamykosen, oder Akne ist, vorzugsweise wobei die Emulsion zu einem Akne-Stick verarbeitet ist.

11. Emulsion gemäß einem der Ansprüche 1 bis 5 zur Anwendung in der topischen Behandlung von Blasenentzündungen der Frau sowie zur lokalen Partnerbehandlung (Glans penis, Anfangsdrittel der Urethra).

12. Emulsion vorzugsweise Shampoos gemäß einer der Ansprüche 1 bis 5 zur Behandlung von Haarausfall.

13. Emulsion gemäß einem der Ansprüche 3 bis 5, zur Anwendung in der topischen Behandlung von atopischer Dermatitis.

14. Ein Verfahren zur Herstellung einer Emulsion gemäß einer der Ansprüche 1 bis 5, **dadurch gekennzeichnet, dass** bei der Herstellung der Emulsion das NSAID über die wässrige Phase eingebracht wird.

15. Ein Verfahren zur Herstellung einer Emulsion gemäß einer der Ansprüche 2 bis 5, **dadurch gekennzeichnet, dass** das NSAID als feinkristallines oder mikronisiertes Salz in die das Antimykotikum enthaltene Emulsion eingebracht wird.

## Claims

1. An emulsion for topical treating of dermal infections and mucosal infections, particularly urogenital infectious diseases, **characterized in that** an antimicrobial agent selected from an antibiotic and an antiseptic and an NSAID are used in combination, wherein (a) the NSAID is diclofenac in a concentration of 0.1 to 0.5 weight percent, indometacin in a concentration of 0.1 to 0.4 weight percent, naproxen in a concentration of 1 to 5 weight percent, ibuprofen in a concentration of 0.5 to 2.5 weight percent, dexibuprofen in a concentration of 0.25 to 1.25 weight percent, ketoprofen in a concentration of 0.25 to 1.25 weight percent, mefenamic acid in a concentration of 0.5 to 4 weight percent, or lornixocam in a concentration of 0.02 to 0.04 weight percent, wherein the NSAID is in salt form; (b) the weight ratio of the water to the oil phase in said emulsion is between the values 2.0 and 2.7, wherein the weight ratio is calculated including the fabrics dissolved in the phases, wherein emulsifiers are not included in either the water or the oil phase, and (c) the pH of the emulsion is not less than the value 6.5 and not more than 8.5, preferably in the area 7.0 to 8.

2. An emulsion according to claim 1, which additionally contains an antifungal agent.

3. An emulsion according to claim 1 or 2, in the form of an ointment, a cream, a shampoo or a stick, preferably for topical treating of dermal infections, preferably further containing an odorant, preferably a terpene, particularly farnesol.

4. An emulsion according to any of claims 1 to 3, **characterized in that** the antimicrobial agent is an antibiotic, preferably phosphomycin, clindamycin, metronidazole, nitrofurantoin, nitrofurazone, nitrofurantoin, nifuratel, nifuroxacin, nitroxoline, trimethoprim, sulfadiazine, or cotrimoxazole; or that the antimicrobial agent is an antiseptic, preferably selected from the group consisting of: Benzalkonium chloride, preferably in a concentration of at least 0.2 weight percent; Dequalinium chloride, preferably in a concentration of at least 0.2 weight percent; and Phenoxyethanol, preferably in a concentration of at least 2 weight percent.

5. An emulsion according in accordance with any of claims 2 to 4, **characterized in that** the antifungal agent is nystatin, ciclopirox or ciclopiroxolamine, or an antifungal agent from the group of azoles, preferably clotrimazole, fluconazole, miconazole, itraconazole, tioconazole, voriconazole, bifonazole, econazole, isoconazole, fenticonazole, sertaconazole, ketoconazole, posaconazole, quilseconazole, oteseconazole (VT-1161) or ibrexafungerp (SCY-078).

6. An emulsion according to any of claims 1 to 5 for use in the topical treating of infectious diseases, in particular for use in the topical treating of dermal and urogenital infectious diseases, preferably wherein the infectious disease is a chronic infectious disease.

7. The emulsion for use according to claim 6, **characterized in that** the infectious disease is a mucosal infection, in particular a urogenital infection, preferably a microbial urogenital infection, in particular a female microbial urogenital infection, preferably wherein the microbial urogenital infection is a bacterial urogenital infection.

8. The emulsion for use according to claim 6 or 7, **characterized in that** the infectious disease is a mixed vaginal infection by Candida albicans and bacteria such as Enterobacter, E.coli, Klebsiella pneumoniae, Gardnerella vaginalis, Prevotella spp.

9. The emulsion for use according to any of claims 6 to 8,
**characterized in that** the infectious disease is an asymptomatic or symptomatic bacterial vaginosis or an asymptomatic or symptomatic dysbiosis of the glans penis and/or the male urethra.

10. The emulsion for use according to claim 9, **characterized in that** the infectious disease is a dermal fungal infection, preferably selected from candidamycoses and malasseziamycoses, or acne, preferably wherein the emulsion is processed into an acne stick.

11. The emulsion according to any of claims 1 to 5 for use in the topical treating of female cystitis and for local partner treatment (glans penis, initial third of the urethra).

12. The emulsion, preferably shampoos according to any of claims 1 to 5, for treating hair loss.

13. The emulsion in accordance with any of claims 3 to 5, for use in the topical treating of atopic dermatitis.

14. A method of producing an emulsion according to any of claims 1 to 5, **characterized in that** in the production of the emulsion the NSAID is introduced via the aqueous phase.

15. A method for the production of an emulsion according to any of claims 2 to 5, **characterized in that** the NSAID is incorporated as a fine crystalline or micronized salt into the emulsion containing the antifungal agent.

## Revendications

1. Emulsion pour le traitement topique des infections dermiques et des infections des muqueuses, en particulier des maladies infectieuses urogénitales, **caractérisée en ce qu'**un principe actif antimicrobien choisi parmi un antibiotique et un antiseptique, et un AINS sont utilisés en combinaison, où (a) l'AINS est le diclofénac à une concentration de 0,1 à 0,5 % en poids, l'indométhacine à une concentration de 0,1 à 0,4 % en poids, le naproxène à une concentration de 1 à 5 % en poids, l'ibuprofène à une concentration de 0,5 à 2,5 % en poids, le dexibuprofène à une concentration de 0,25 à 1,25 % en poids, le kétoprofène à une concentration de 0,25 à 1,25 % en poids, l'acide méfénamique à une concentration de 0,5 à 4 % en poids ou le lornixocam à une concentration de 0,02 à 0,04 % en poids, où l'AINS est sous forme de sel; (b) le rapport pondéral de la phase aqueuse à la phase huileuse dans cette émulsion est situé entre les valeurs 2,0 et 2,7, où le rapport pondéral est calculé en tenant compte des substances dissoutes dans les phases, où des émulsifiants ne sont comptés ni dans la phase aqueuse ni dans la phase huileuse, et (c) le pH de l'émulsion n'est pas inférieur à la valeur 6,5 et pas supérieur à 8,5, de préférence dans la gamme de 7,0 à 8.

2. Emulsion selon la revendication 1, qui contient en outre un principe actif antimycosique.

3. Emulsion selon la revendication 1 ou 2, sous forme d'une pommade, d'une crème, d'un shampoing ou d'un stick, de préférence pour le traitement topique des infections dermiques, de préférence contenant en outre un agent odorant, de préférence un terpène, en particulier le farnésol.

4. Emulsion selon l'une des revendications 1 à 3, **caractérisée en ce que** le principe actif antimicrobien est un antibiotique, de préférence la phosphomycine, la clindamycine, le métronidazole, la nitrofurantoïne, la nitrofurazone, la nitrofurantoïne, le nifuratel, la nifuroxacine, la nitroxoline, le triméthoprime, la sulfadiazine ou le cotrimoxazole; ou **en ce que** le principe actif antimicrobien est un antiseptique, de préférence choisi dans le groupe consistant en: le chlorure de benzalkonium, de préférence à une concentration d'au moins 0,2 % en poids; le chlorure de déqualinium, de préférence à une concentration d'au moins 0,2 % en poids; et le phénoxyéthanol, de préférence à une concentration d'au moins 2 % en poids.

5. Emulsion selon l'une des revendications 2 à 4, **caractérisée en ce que** le principe actif antimycosique est la nystatine, le ciclopirox ou la ciclopiroxolamine, ou un antimycosique du groupe des azoles, de préférence le clotrimazole, le fluconazole, le miconazole, l'itraconazole, le tioconazole, le voriconazole, le bifonazole, l'éconazole, l'isoconazole, le fenticonazole, le sertaconazole, le kétoconazole, le posaconazole, le quilséconazole, l'otéséconazole (VT-1161) ou l'ibrexafungerp (SCY-078).

6. Emulsion selon l'une des revendications 1 à 5 pour une utilisation dans le traitement topique des maladies infectieuses, en particulier pour une utilisation dans le traitement topique des maladies infectieuses dermiques et urogénitales, de préférence où la maladie infectieuse est une maladie infectieuse chronique.

7. Emulsion pour une utilisation selon la revendication 6, **caractérisée en ce que** la maladie infectieuse est une infection des muqueuses, en particulier une infection urogénitale, de préférence une infection urogénitale microbienne, en particulier une infection urogénitale microbienne de la femme, de préférence où l'infection urogénitale microbienne est une infection urogénitale bactérienne.

8. Emulsion pour une utilisation selon la revendication 6 ou 7, **caractérisée en ce que** la maladie infectieuse est une infection vaginale mixte par Candida albicans et des bactéries telles que Enterobacter, E. coli, Klebsiella pneumoniae, Gardnerella vaginalis, Prevotella spp..

9. Emulsion pour une utilisation selon l'une des revendications 6 à 8, **caractérisée en ce que** la maladie infectieuse est une vaginose bactérienne asymptomatique ou symptomatique ou une dysbiose asymptomatique ou symptomatique du gland du pénis et/ou de l'urètre masculin.

10. Emulsion pour une utilisation selon la revendication 9, **caractérisée en ce que** la maladie infectieuse est une infection fongique dermique, de préférence choisie parmi les mycoses à Candida et les mycoses à Malassezia, ou l'acné, de préférence où l'émulsion est mise sous forme de stick anti-acné.

11. Emulsion selon l'une des revendications 1 à 5 pour une utilisation dans le traitement topique des inflammations de la vessie chez la femme ainsi que pour le traitement local du partenaire (gland du pénis, premier tiers de l'urètre).

12. Emulsion de préférence shampooings selon l'une des revendications 1 à 5 pour le traitement de la chute des cheveux.

13. Emulsion selon l'une des revendications 3 à 5, pour une utilisation dans le traitement topique de la dermatite atopique.

14. Procédé de préparation d'une émulsion selon l'une des revendications 1 à 5, **caractérisé en ce que** l'AINS est introduit par le biais de la phase aqueuse lors de la préparation de l'émulsion.

15. Procédé de préparation d'une émulsion selon l'une des revendications 2 à 5, **caractérisé en ce que** l'AINS est introduit dans l'émulsion contenant l'antimycosique sous forme de sel finement cristallin ou micronisé.
